(19) **Europäisches Patentamt**
**European Patent Office**
**Office européen des brevets**

(11) **EP 2 191 004 B1**

(12) **EUROPEAN PATENT SPECIFICATION**

(45) Date of publication and mention of the grant of the patent:
**19.06.2013 Bulletin 2013/25**

(51) Int Cl.:
*C12P 7/56* (2006.01)  *C12P 7/40* (2006.01)
*C12N 1/22* (2006.01)  *C12M 1/36* (2006.01)

(21) Application number: **08793837.9**

(22) Date of filing: **12.08.2008**

(86) International application number:
**PCT/NL2008/050545**

(87) International publication number:
**WO 2009/025547 (26.02.2009 Gazette 2009/09)**

(54) **MILD ALKALINE PRETREATMENT AND SIMULTANEOUS SACCHARIFICATION AND FERMENTATION OF LIGNOCELLULOSIC BIOMASS INTO ORGANIC ACIDS**

MILDE ALKALISCHE VORBEHANDLUNG UND GLEICHZEITIGE VERZUCKERUNG UND VERGÄRUNG VON LIGNOCELLULOSE-BIOMASSE ZU ORGANISCHEN SÄUREN

PRÉTRAITEMENT ALCALIN DOUX ET SACCHARIFICATION ET FERMENTATION SIMULTANÉES DE BIOMASSE LIGNOCELLULOSIQUE EN ACIDES ORGANIQUES

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **23.08.2007 EP 07114875**

(43) Date of publication of application:
**02.06.2010 Bulletin 2010/22**

(73) Proprietor: **Stichting Dienst Landbouwkundig Onderzoek**
**6708 PB Wageningen (NL)**

(72) Inventors:
• **BAKKER, Robert Reurd Christophor**
**NL-6708 KR Wageningen (NL)**
• **DE JONG, Ed**
**NL-6703 BR Wageningen (NL)**
• **MAAS, Ronald Hubertus Wilhelmus**
**NL-6871 TN Renkum (NL)**
• **WEUSTHUIS, Ruud Alexander**
**NL-6866 GD Heelsum (NL)**
• **VISSER, Diana**
**NL-4201 EP Gorinchem (NL)**
• **WINKELAAR, Hendrik Martinus**
**NL- 4927 BP Hoge Zwaluwe (NL)**
• **JANSEN, Mickel Leonardus August**
**NL-2562 XN Den Haag (NL)**

(74) Representative: **Swinkels, Bart Willem**
**Nederlandsch Octrooibureau**
**J. W. Frisolaan 13**
**2517 JS Den Haag (NL)**

(56) References cited:
**WO-A-94/13826       WO-A-2005/086670**
**US-A1- 2006 014 260**

• LIU ET AL: "Process of rice straw degradation and dynamic trend of pH by the microbial community MC1" JOURNAL OF ENVIRONMENTAL SCIENCES, vol. 18, no. 6, November 2006 (2006-11), pages 1142-1146, XP005796579
• OHKOUCHI ET AL: "Direct production of l (+)-lactic acid from starch and food wastes using Lactobacillus manihotivorans LMG18011" BIORESOURCE TECHNOLOGY, vol. 97, no. 13, September 2006 (2006-09), pages 1554-1562, XP005456179 ISSN: 0960-8524
• ROY S ET AL: "Optimal control strategies for simultaneous saccharification and fermentation of starch." PROCESS BIOCHEMISTRY, vol. 36, no. 8/9, 2001, pages 713-722, XP002464994
• CHEN RONGFU ET AL: "Membrane-mediated extractive fermentation for lactic acid production from cellulosic biomass" APPLIED BIOCHEMISTRY AND BIOTECHNOLOGY, vol. 63-65, no. 0, 1997, pages 435-448, XP008087560 ISSN: 0273-2289

**(Cont. next page)**

- BORDEN J R ET AL: "Simultaneous saccharification and fermentation of cellulosic biomass to acetic acid." APPLIED BIOCHEMISTRY AND BIOTECHNOLOGY, vol. 84-86, April 2000 (2000-04), pages 963-970, XP008087559 ISSN: 0273-2289
- MOSIER N ET AL: "Features of promising technologies for pretreatment of lignocellulosic biomass" BIORESOURCE TECHNOLOGY, vol. 96, no. 6, 29 September 2004 (2004-09-29), pages 673-686, XP002395515 ISSN: 0960-8524 cited in the application
- CHANG V S ET AL: "Lime pretreatment of crop residues bagasse and wheat straw" APPLIED BIOCHEMISTRY AND BIOTECHNOLOGY, vol. 74, no. 3, September 1998 (1998-09), pages 135-159, XP008087461 ISSN: 0273-2289 cited in the application
- KAAR W E ET AL: "Using lime pretreatment to facilitate the enzymic hydrolysis of corn stover" BIOMASS AND BIOENERGY, vol. 18, no. 3, 2000, pages 189-199, XP002464996 ISSN: 0961-9534 cited in the application
- AKAO S ET AL: "Semi-continuous l-lactate fermentation of garbage without sterile condition and analysis of the microbial structure" WATER RESEARCH, vol. 41, no. 8, 23 March 2007 (2007-03-23), pages 1774-1780, XP005935305 ISSN: 0043-1354
- PATEL M A ET AL: "Simultaneous Saccharification and Co-Fermentation of Crystalline Cellulose and Sugar Cane Bagasse Hemicullose Hydrolysate to Lactate by a Thermotolerant Acidophilic Bacillus sp" BIOTECHNOLOGY PROGRESS, vol. 21, 1 January 2005 (2005-01-01), pages 1453-1460, XP003009606 ISSN: 8756-7938
- PATEL M A ET AL: "Isolation and characterization of acid-tolerant, thermophilic bacteria for effective fermentation of biomass-derived sugars to lactic acid" APPLIED AND ENVIRONMENTAL MICROBIOLOGY, vol. 72, no. 5, May 2006 (2006-05), pages 3228-3235, XP008067807 ISSN: 0099-2240
- ZHANG ET AL: "Production of lactic acid from renewable materials by Rhizopus fungi" BIOCHEMICAL ENGINEERING JOURNAL, vol. 35, no. 3, 31 May 2007 (2007-05-31), pages 251-263, XP022100388 ISSN: 1369-703X
- JOHN R P ET AL: "Fermentative production of lactic acid from biomass: an overview on process developments and future perspectives" APPLIED MICROBIOLOGY AND BIOTECHNOLOGY, vol. 74, no. 3, March 2007 (2007-03), pages 524-534, XP002464997 ISSN: 0175-7598
- HOFVENDAHL KARIN ET AL: "Factors affecting the fermentative lactic acid production from renewable resources" ENZYME AND MICROBIAL TECHNOLOGY, vol. 26, no. 2-4, February 2000 (2000-02), pages 87-107, XP002500788 ISSN: 0141-0229
- "Current Developments in Solid-state Fermentation" 2008, CURRENT DEVELOPMENTS IN SOLID-STATE FERMENTATION SPRINGER, 233 SPRING STREET, NEW YORK, NY 10013, UNITED STATES , XP002500778 ISSN: 978-0-387-75212-9 (H) chapter 10, pages 205-229 Soccol C R et al., Production of organic acids by solid-state fermentation

**Description**

Field of the invention

The invention relates to a method for the production of organic acids as a fermentation product from lignocellulosic biomass, wherein the lignocellulosic biomass is pretreated using an alkaline agent. The invention further relates to a reactor to carry out the method of the invention.

Background of the invention

Lignocellulosic biomass feedstocks may be used for fermentation processes, in particular, bioethanol and lactic acid. Conventional processes for converting lignocellulosic materials into bulk chemicals, such as lactic acid, requires pretreatment, enzymatic hydrolysis and microbial fermentation. Lignocellulosic biomass is an inexpensive and widely available renewable carbon source that has no competing food value. Lignocellulose consists primarily of cellulose and hemicellulose; polymers build up of mainly hexose sugars and pentose sugars, which are embedded in a matrix of the phenolic polymer lignin. The main pathway to derive fermentable sugars from lignocellulose is through enzymatic hydrolysis by cellulolytic and hemicellulolytic enzymes. A mechanical and chemical pretreatment of the lignocellulose is required in order to reduce particle size, to modify and/or to remove the lignin and with that enhance the accessibility of the polysaccharides for enzymatic hydrolysis (Claassen et al. (1999) Microbiol. Biotechnol. 52:741-755).

Various chemical pretreatments of biomass have been studied in research and development of lignocellulose-to-ethanol production technology (Mosier et al. (2005) Bioresour. Technol. 96:673-686). Alkaline pretreatment of lignocellulosic biomass with lime ($Ca(OH)_2$) at mild temperatures (<100°C) was shown to be a promising pretreatment route to enhance enzymatic hydrolysis, and can be characterized by high enzymatic degradability with no significant delignification or xylan degradation of the lignocellulosic substrate (Chang et al. (1998) Appl. Biochem. Biotechnol. 74:135-159). Nevertheless, this alkaline pretreatment at a relatively high pH value (>10) is not attractive since the activity of common cellulolytic and xylanolytic enzymes, necessary for the depolymerization of (hemi)-cellulose, is low at this pH. For example, Bacillus sp. strain 36D1 was disclosed to be a biocatalyst for lactic acid production via simultaneous saccharification and fermentation (SSF) in which sugars derived from both hemicellulose and cellulose are fermented in a single vessel and wherein the volumetric productivity of SSF to lactic acid was optimal with pH 5.0 at 50°C (Patel et al. Biotechnol. Prog. 2005, 21, 1453-1460).Therefore, lowering the pH is essential in order to achieve an efficient enzymatic hydrolysis of the polysaccharides. One approach to remove calcium hydroxide is by washing the lime-treated biomass prior to enzymatic hydrolysis (Chang et al. (1998) Appl. Biochem. Biotechnol. 74:135-159); however, this leads to the use of high amounts of water. Another way to lower the pH of the pretreated material is by neutralizing calcium hydroxide with acids, such as sulphuric acid and acetic acid, or with $CO_2$. Yet this results in the formation of the low value salts as by-product, such as gypsum or calcium carbonate. Therefore, this problem of high pH value (>10) of lignocellulosic material after pretreatment with lime and prior to enzymatic hydrolysis, has not yet been properly solved.

The present invention provides a method for the production of an organic acid as a fermentation product from lignocellulosic biomass, a reactor to carry out the method of the invention and use of the reactor to carry out the method of the invention, wherein the alkaline nature of the alkaline pretreated biomass is used in the simultaneous saccharification and fermentation process in order to control the pH during fermentation.

Description of the invention

In one aspect, the present invention provides a method for the production of an organic acid as a fermentation product from lignocellulosic biomass, comprising the steps of:

a) Pretreatment of lignocellulosic biomass with an alkaline agent to obtain alkaline pretreated lignocellulosic biomass with a pH of between about 8.0 and about 14.0;
b) Simultaneous saccharification and fermentation (SSF) of the alkaline pretreated lignocellulosic biomass of step a) in a fermentor, whereby the decrease in pH, caused by the production of the organic acid, is counter acted by the addition of alkaline pretreated lignocellulosic biomass as obtained in step a) with a pH of between about 8.0 and about 14.0, optionally in combination with an alkali, to adapt the pH below about 8.0 and/or to maintain the pH at a specific pH below 8.0, allowing optimal activity of the micro-organism(s) and/or enzymes added; and
c) Optionally recovery of the fermentation product; wherein the SSF of step b) comprises a pre-hydrolysis phase wherein the pH of a part of the alkaline pretreated biomass obtained by step a) is adapted by addition of one or more acids to between 2.0 and 8.0 to allow for conversion of alkaline pretreated biomass into fermentable sugars to start the microbial conversion of biomass into organic acids.

The term "organic acid as a fermentation product" is herein defined as a product that has been obtained by fermentation by one or more microorganisms, in which the product is an organic molecule comprising at least one carboxy group. In one embodiment, the fermentation product that is produced by the method of the present invention is selected from the group consisting of, but not limited to: lactic acid, citric acid, itaconic acid, succinic acid, fumaric acid, glycolic acid, pyruvic acid, acetic acid, glutamic acid, malic acid, maleic acid, propionic acid, butyric acid, gluconic acid and combinations thereof. In a particularly preferred embodiment, the fermentation product is lactid acid.

Alternatively or in combination with previous preferred embodiments, in a further preferred embodiment, the lignocellulosic biomass is selected from the group consisting of, but not limited to: grass, wood, bagasse, straw, paper, plant material (straw, hay, etc.), and combinations thereof. In one embodiment, the lignocellulosic biomass is wheat straw, maize straw, barley straw, rice straw, rye straw or straw from any cultivated plant.

Alternatively or in combination with previous preferred embodiments, in a further embodiment, the lignocellulosic biomass is air dry, having at least 50, 55, 60, 65, 70, 75, 80, 85, 89.5, 90 or 95% (w/w) dry matter. In another embodiment, the lignocellulosic biomass is not dried, i.e. fresh biomass may be used.

Alternatively or in combination with previous preferred embodiments, in a further preferred embodiment, the lignocellulosic biomass undergoes a pre-extraction prior to pretreatment in order to remove non-fermentable soluble components such as proteins, amino acids or soluble inorganic components contained in the biomass which may interfere with subsequent hydrolysis and fermentation. In another preferred embodiment, fermentable soluble components may be removed from the lignocellulosic biomass. The term "pre-extraction" is herein defined as any treatment removing soluble components from the lignocellulosic biomass.

Alternatively or in combination with previous preferred embodiments, in a further preferred embodiment, the pretreatment of lignocellulosic biomass is preceded by or combined and/or integrated with a mechanical comminution of lignocellulosic biomass. Mechanical comminution is performed in order to change the particle size distribution of the lignocellulosic biomass in such a way that the efficiency of the pretreatment and subsequent processes are improved, and that the alkaline agent is thoroughly mixed into the lignocellulosic biomass. In a preferred embodiment, mechanical comminution comprises, but is not limited to: milling, mechanical refining and extrusion.

Step a) Pretreatment of lignocellulosic biomass with an alkaline agent

Pretreatment of lignocellulosic biomass is required in order to break open the lignocellulosic matrix, removing or modifying lignin and increasing the surface area of cellulose. The term "pretreatment" is herein defined as any method performed before hydrolysis aiming to increase the degree of hydrolysis after hydrolysation of lignocellulose. Lignocellulosic biomass pretreatment is preferably carried out until at least about 50%, more preferably at least about 75%, yet more preferably at least about 85% of carbohydrate components in the pretreated biomass are converted by one or more hydrolytic enzyme(s) into one or more monomeric sugars within a reasonable period of time, such as about 24 hours.

In combination with previous preferred embodiments, in a further preferred embodiment, the present invention relates to pretreatment of lignocellulosic biomass with an alkaline agent to obtain alkaline pretreated lignocellulosic biomass with a pH ranged between about 8.0 and about 14.0, preferably between about pH 8.0 and pH 12.5 or 12.0. Thus, a suitable amount of one or more alkaline agents are added to the biomass and incubated for a suitable period of time and at a suitable temperature, as indicated herein below. During alkaline pre-treatment of the lignocellulosic biomass, the pH may decline about 0.5 to about 2.0 units due to release of acids contained in the lignocellulosic biomass. In a preferred embodiment, the alkaline pretreated lignocellulosic biomass according to the invention has a pH value ranged between about 8.5 and about 12.5, more preferably ranged between about 9.0 and about 12.0, even more preferably ranged between about 9.5 and about 12.0, most preferably a pH value of about 11.8.

Alternatively or in combination with previous preferred embodiments, in a further preferred embodiment, the alkaline agent to be used in step a) of the method of the present invention is selected from the group consisting of, but not limited to: calcium hydroxide ($Ca(OH)_2$), calcium oxide (CaO), ammonia ($NH_3$), sodium hydroxide (NaOH), potassium hydroxide (KOH), urea, or combinations thereof.

Alternatively or in combination with previous preferred embodiment, in a further preferred embodiment, the alkaline agent:lignocellulosic biomass ratio is ranged between about 1:100 and about 20:100, more preferably between about 2.5:100 and about 17.5:100 and most preferably between about 5:100 and about 15:100. The alkaline agent: lignocellulosic biomass ratio may be selected in such a way as to improve the enzymatic degradability and fermentability of cellulose and hemicellulose. Alternatively or in combination with previous preferred embodiment, in a further preferred embodiment, the pretreatment of lignocellulosic biomass with an alkaline agent is carried out at a suitable temperature. The most suitable temperature for carrying out step (a) of the invention is the temperature resulting in the lowest production costs of the fermentation product, preferably without affecting the fermentation efficiency, for any selected type and concentration of biomass, the selected other conditions of pretreatment (e.g., pH and time period) and the selected conditions for SSF (e.g., microorganism(s), temperature, enzyme(s)). In a preferred embodiment the suitable temperature is ranged between about 50°C and about 115°C, more preferably between about 60°C and about 95°C, more preferably between about 70°C and about 90°C, even more preferably between about 80°C and about 90°C and most preferably about 85°C.

Alternatively or in combination with previous preferred embodiment, in a further preferred embodiment, the pretreatment of lignocellulosic biomass with an alkaline agent is carried out for a time period ranged between about 2 hours to about 20 hours, more preferably between about 4 hours and about 16 hours, more preferably between about 5 hours and about 12 hours, more preferably between about 6 hours and about 10 hours, and most preferably for a time period of about 8 hours. The most suitable time period for carrying out step (a) of the invention is the time period, resulting in the lowest production costs of the fermentation product, preferably without affecting the fermentation efficiency, for any selected type and concentration of biomass, the selected other conditions of pretreatment (e.g., pH and temperature) and the selected conditions for SSF (e.g., microorganism(s), temperature, enzyme(s)).

Alternatively or in combination with previous preferred embodiment, in a further preferred embodiment, the alkaline pretreated lignocellulosic biomass of the invention, is subjected to one or more of the following steps prior to SSF: a cooling step, a washing step and/or a dewatering step. In a more preferred embodiment, the alkaline pretreated lignocellulosic biomass is cooled to about 80°C, more preferably to about 70°C, more preferably to about 60°, more preferably to about 50°C, more preferably to about 40°C and most preferably about 30°C. The term "dewatering" or "dehydration" as used herein is defined as removing free water from the biomass. In another preferred embodiment, the dewatering step is performed by using filtration while applying pressure to the pretreated biomass, wherein the applied pressure is ranged between 0 and about 100 bar. Other methods of dewatering will be known to the person skilled in the art. In another more preferred embodiment, the washing step is performed to remove fermentation inhibitors such as organic acids (*e.g.*, acetic acid). In a preferred embodiment, the washing step is performed by addition of water after dewatering followed by a next dewatering step.

Alternatively or in combination with a previous preferred embodiment, in a further preferred embodiment of the present invention, the alkaline pretreated lignocellulosic biomass of step (a) is added to the simultaneous saccharification and fermentation (SSF) process of step (b) described below, more preferably in a fed-batch manner, in order to neutralize the acidification which is caused by the microbial fermentation in step (b). The term "neutralize" or "neutralisation" is herein defmed as adapting and/or maintaining the pH of the SSF mixture to a pH equal to or below about 8.0, such as adapting and/or maintaining the pH to/at a specific pH of about 4.5, 5.0, 5.5, 6.0, 6.5, 7.0, 7.5 or 8.0, depending on pH optima of hydrolytic enzyme(s) and/or microorganism(s). Thus, in the SSF process of step (b) described below, alkaline pretreated lignocellulosic biomass is added to the SSF mixture to counterbalance the pH decrease caused by the production of the organic acid, maintaining the pH at a constant level. The person skilled in the art will know how to choose the pH level to be maintained in the SSF mixture.

Step b) Simultaneous saccharification and fermentation

The term "simultaneous saccharification and fermentation" (SSF) is herein defined as the simultaneous enzymatic hydrolysis of polymeric carbohydrates of the alkaline pretreated lignocellulosic biomass into fermentable saccharides and the further conversion of saccharides into the fermentation product by one or more microorganism(s).

The present invention relates to SSF of the alkaline pretreated lignocellulosic biomass in a fermentor, whereby the alkaline pretreated lignocellulosic biomass is added to the SSF mixture, preferably in a fed-batch manner in order to neutralize the acidification which is caused by the microbial fermentation in step (b).

Alternatively or in combination with a previous preferred embodiment, in a further preferred embodiment of the present invention, the SSF process of step (b) is operated in a chemostat mode, in which the alkaline pretreated lignocellulosic biomass is used as a nutrient. A "chemostat mode" is herein defined as a fermentor device to keep fermentation parameters, such as nutrient concentration and pH, essentially constant. Alternatively or in combination with previous preferred embodiment, in a further preferred embodiment, the SSF comprises the steps of: i) Optionally a pre-hydrolysis phase; ii) Enzymatic hydrolysis with an hydrolytic enzyme to obtain fermentable saccharides; and iii) Microbial fermentation using one or more microorganism(s) which is able to convert the saccharides of step ii) into the fermentation product.

The SSF comprises a pre-hydrolysis phase wherein a part of the alkaline pretreated biomass, of which the pH is adapted to the desired level by addition of one or more acids, is converted into fermentable sugars, providing a suitable environment for the microorganism(s) to start the microbial conversion of biomass into organic acids.

Alternatively or in combination with previous preferred embodiment, in a further preferred embodiment, the pre-hydrolysis phase is performed for a period sufficient to increase the fermentable sugar concentration in the reactor to a value of between 0.5 and 10, more preferably of between 1 and 5 g/l.

Alternatively or in combination with previous preferred embodiment, in a further preferred embodiment, the SSF comprises an enzymatic hydrolysis phase with one or more hydrolytic enzyme preparations to obtain saccharides. The saccharides that may be obtained by enzymatic hydrolysis may comprise e.g. glucose, mannose, fructose, lactose, galactose, rhamnose, xylose, arabinose, galacturonic acid and oligomeric saccharides of (combinations of) these.

Enzymatic hydrolysis of polymeric carbohydrates according to the method of the invention is necessary for formation of a substrate that may be used by the microorganism(s) in the fermentation step. A hydrolytic enzyme for use in the method of the invention, i.e. which is added in a suitable amount to the SSF process of step (b), may be, but is not limited to, the group comprising: cellulase preparations, hemicellulase preparations, cellobiase, xylanase preparations, amylase and pectinase. Such enzyme preparations are commercially available and can for example be obtained from Genencor International B.V. (Leiden, The Netherlands). A suitable amount of hydrolytic enzyme activity for use in the method of the invention is the amount of hydrolytic enzyme activity, resulting in the lowest production costs of the fermentation product while retaining good or optimal enzymatic activity, with the selected type and concentration of biomass, the selected conditions of pretreatment (e.g., pH, time period and temperature) and the selected conditions for SSF (e.g., microorganism(s) and temperature). A suitable amount of hydrolytic enzyme activity would for example be within the range of about 0.01 to about 50, more preferably about 5 to about 40 Filtre paper units (FPU), although higher hydrolytic enzyme activities may be used. Alternatively or in combination with previous preferred embodiment, in a further preferred embodiment, the enzymatic hydrolysis during SSF according to the method of the invention yields a hydrolysate that comprises very low concentrations or even negligible concentrations of fermentation inhibiting compounds. In a preferred embodiment, the concentrations of furfural, 5-hmf and phenolic compounds that are formed in the SSF are less than 0.2 g/l. The amount of such compounds can be measured using standard methods, such as HPLC analysis.

The one or more microorganism(s) for use in the method of the invention, i.e. which are added to the SSF mixture of step (b) above before and/or during SSF, may be a bacterium, a fungus (including a yeast), an archaea or an algae. In a preferred embodiment, the bacterium is selected from a thermotolerant *Bacillus* strain. In another preferred embodiment, the microorganism is selected from the group consisting of, but not limited to: *Acetobacter aceti, A. hansenii, A. liquefaciens, A. mesoxydans, A. pasteurianus, A. suboxydans, A. xylinum, Achromobacter agile, A. lactium, Acinetobacter baumanii, A. calcoaceticus, A. genospecies, A. genospesis, A. haemolyticus, A. junii, Acinetobacter sp. Actinomycete sp., Actinoplane missouriensis, Aerobacter aerogenes, A. cloacae, Aeromonas culicicola, A. formicans, Aeromonas sp., Agrobacterium radiobacter, A. rhizogenes, A. tumefaciens, Alcaligenes faecalis, Alcaligenes sp., A. tolerans, A. viscolactis, Amylolatopsis mediterranei, Anabaena ambigua, A. subcylindria, Aquaspirillium intersonii, Arthroascus javanensis, Arthrobacter albidus, A. citreus, A. luteus, A. nicotinae, A. polychromogenes, A. simplex, Arthrobacter sp., A. ureafaecalis, A. viscosus, Azomonas macrocytogenes, Azospirillum brasilense, A. lipoferum, Azotobacter chroococcum, A. agilis, A. chroococcum, A. macrocytogenes, Azotobacter sp., A. vinelandii, Azotomonas insolita, Bacillus aminovorans, B. amyloliquefaciens, B. aneurinolyticus, B. aporrheous, B. brevis, B. cereus, B. cereus subsp. mycoids, B. circulans, B. coagulans, B. firmus, B. freudenreichii, B. globigii, B. laevolacticus, B. laterosporus, B. lentus, B. licheniformis, B. macerans, B. macquariensis, B. marcescens, B. megaterium, B. mesentericus, B. pantothenticus, B. pasteurii, B. polymyxa, B. pumilus, B. racemilacticus, Bacillus sp., B. sphaericus, B. stearothermophilus, B. subtilis, B. thuringiensis, B. zopfii, B. subtilis, Beijerinckia indica, B. lactiogenes, Bordetella bronchiseptica, Brettanomyces intermedius, Brevibacterium ammoniagene, B. diverticatum, B. immariophilum, B. imperiale, B. linens, B. liquifaciens, B. luteum, B. roseum, B. saccharolyticum, B. vitarumen, Candida albicans, C. bombii, C. brumptii, C. catenulata, C. colliculosa, C. deformans, C. epicola, C. etchellsii, C. famata, C. freyschussii, C. glabrata, C. gropengiesseri, C. guilliermondii, C. krusei, C. lambica, C. lusitaniae, C. magnoliae, C. mannitofaciens, C. melibiosica, C. mucifera, C. parapsilosis, C. pseudotropicalis, C. rugosa, C, rugosa, C. tropicalis, C. utilis, C. versatilis, C. wickerhamii, C. sake, C. shehatae Candida Sp., C. stellata, Cellulomonas bibula, C. bizotea, C. cartae, C. fimi, C. flavigena, C. gelida, C. uda, Chainia sp., Chlorella pyrenoidosa, Chromatium sp., Citeromyces matritensis, Citrobacter fruendii, C. acetobutylicum, C. felsineum, C. pasteurianum, C. perfringens, C. roseum, C. sporogenes, C. tetanomorphum, Corynebacterium rubrum, Corynebacterium glutamicum, Corynebacterium sp., Cryptococcus laurentii, C. leteolus, C. neoformans, C. neoformans, Crytococcus sp., Cytophaga hutchinsonii, Debaryomyces castellii, D. fibuligera, D. hansenii, D. marama, D. polymorphus, D. vanriji, Dekerra anomala, D. claussenii, D. bruxellensis, D. intermedia, D. naardensis, Desulfotomaculum nigrificans, Desulfovibrio desulfuricans, Enterobacter aerogenes, E. clocae, Erwinia cherysanthemi, Escherichia coli, E. intermedia, E. irregular, Euglena gracilis, Filobasidium capsuligenum, F. uniguttulatum, Flavobacterium dehydrogenans, F. devorans, F. odoratum, Flavobacterium sp., Geotrichum sp., Gluconobacter melanogenes, G. melanogenus, G. oxydans, G. roseus, Guilliermondella selenospora, Hafnia alvei, Halobacterium cutirubrum, H. halobium, H. salinarium, H. trapinium, Haneseniaspora vineae, Hansenula beckii, H. beijerinckii, H. canadensis, H. capsulata, H. ciferrii, H. polymorpha, H, valbiensis, Hormoascus ambrosiae, Issatchenkia orientalis, Janthinobacter lividum, Jensinia canicruria, Klebsiella aerogenes, K. pneumoniae, K. terrigena, Klockera corticis, K. javancia, Kluveromyces marxianus, Kluyvera citrophila, K. lodderi, K. marxianus, K. marxianus var. lactis, Lactobacillus acidophilus, L. brevis, L. buchneri, L. bulgaricus, L. casei, L. casei var. rhamnosus, L. delbrueckii, L. fermentum, L. helveticus, L. jugurti, L. lactis, L. leichmannii, L. pentosus, L. plantarum, Lactobacillus sp., L. sporogenes, L.viridescens, Leuconostoc mesenteroides, L. oenos, Leuconostoc sp., Leucosporidium frigidium, Lineola longa, Lipomyces lipofera, L. starkeyi, Metschnikowia pulcherrima, M reukaufii, Micrococcus sp., Micrococcus flavus, M glutamicus, M luteus, Microcyclus aquaticus, M flavus, Morexella sp., Mycobacterium phlei, M smegmatis, Mycobacterium sp., Mycoplana bullata, M dimorpha, Mycrocyclus aquaticus, Nadsonia elongata, Nematospora coryli, Nitrobacter sp., Nitrosomonas sp., Nocardia asteroids, N. calcaria, N. cellulans, N. hydrocarbonoxydans, N. mediterranei,*

*N. rugosa, Nocardia sp., Nocardiopsis dassonvillei, Nostoc elipsosporum, N. entrophytum, N. muscorum, N. punctriforme, Oerskovia xanthineolytica, Oosporidium margaritiferum, Pachysolen tannophilus, Pachytichospora transvaalensis, Pediococcus acidilactici, P. cerevisiae, P. pentosaceous, Pichia anomala, P. carsonii, P. farinosa, P. fermentans, P. fluxuum, P. guilliermondii, P. haplophila, P. ohmeri, P. pastoris, P. pijperi, P. rhodanensis, P. toletana, P. trihalophila, P. stipitis, Propionibacterium freudenreichii, P. shermanii, P. thoenii, P. zeae, Protaminobacter alboflavus, Proteus mirabilis, P. morganii, P. morgani, P. vulgaris, Prototheca moriformis, Providencia styartii, Pseudomonas aeruginosa, P. acidovorans, P. aeruginosa, P. aureofaciens, P. auruginosa, P. azotogensis, P. caryophylli, P. cepacia, P. convexa, P. cruciviae, P. denitrificans, P. desmolytica, P. desmolyticum, P. diminuta, P. fluorescens, P. fragi, P. glutaris, P. hydrophila, P. lemonnieri, P. maltophilia, P. mildenbergi, P. oleovorans, P. ovalis, P. pictorum, P. pisi, P. pseudoalcaligenes, P. pseudoflava, P. putida, P. reptilivora, P. resinivorans, P. solanacerum, Pseudomonas sp., P. stutzeri, P. syringae, P. testosteroni, P. viridiflava, Rhizobium indigofera, R. japonicum, R. leguminosarum, R. lupini, R. meliloti, R. phaseoli, Rhizobium sp, R. trifoli, Rhodococcus sp., R. terrae, Rhodosporidium torreloides, Rhodotorula aurantiaca, R. glutinis, R. graminis, R. marina, R. minuta, R. rubra, Rhodotorula sp., Saccharomyces capsulraries, S. cerevisiae, Saccharomycodes ludwigii, Saccharomycopsis fibuligera, Salmonella abony, S. typhimurium, Sarcina lutea, Sarcina sp., S. subflava, Scenedesmus abundans, Schizosaccharomyces octosporus, S. pombe, S. slooffii, Schwanniomyces occidentalis, Serratia marcescens, S. marinorubra, S. plymuthiea, Spirulina sp., Sporobolomyces holsaticus, S. roseus, S. salmonicolor, Sreptomyces diastaticus, S. olivaceous, S. rimosus, Sreptomyces sp., S. venezuelae, Staphylococcus afermentans, S. albus, S. aureus, S. epidermidis, Streptococcus agalactiae, S. cremoris, S. diacetilactis, S. faecalis, S. faecium, S. lactis, S. pyogenes, S. salivaris, Streptococcus sp., S. thermophilus, S. zymogenes, S. peuceticus, S. albogriseolus, S. albus, S. antibioticus, S. atrofaciens, S. aureofaciens, S.caelastis, S. diastaticus, S. erythraeus, S. fluorescens, S. fradiae, S. griseoflavus, S. griseus, S. hawaiiensis, S. hygroscopicus, S. kanamyceticus, S. lavendulae, S. lividans, S. nataliensis, S. nitrosporeus, S. niveus, S. noursei, S. olivaceous, S. olivaceus, S. phacochromogenes, S. pseudogriseolus, Streptomyces sp., S. thermonitrificans, S. venezualae, S. vinaceus, S. viridefaciens, Streptosporangium sp., Streptoverticillium cinnamoneum, S. mobaraense, Streptoverticillium sp., Thermospora sp., Thiobacillus acidophilus, T. ferrooxidans, T. novellus, T. thiooxidans, Torulaspora delbrueckii, Torulopsis ethanolitolerans, T. glabrata, Torulopsis sp., Tremella mesenterica, Trichosporon beigelii, T. capitatum, T. pullulans, Trichosporon sp, Trigonopsis variabilis, Williopsis californica, W. saturnus, Xanthomonas campestris, X malvacearum, Yarrowia lipolytica, Zygosaccharomyces bisporus, Z. rouxii, Z. bisporus, Z. priorionus, Zygosporium aromyces, Z. priorionus, Zymomonas anaerobia, Z. mobilis, Absidia corymbifera, Acremonium chrysogenum, Actinomucor sp., Agaricus bitorquis, Alternaria alternata, A. brassicicola, Alternaria sp., A. terreus, Artrhobotrys conoides, A. oligospora, A. gossypii, Aspergillus awamori, A. candidus, A. clavtus, A. fischeri, A. flavipes, A. flavus, A. foetidus, A. funiculosus, A. luchuensis, A. nidulans, A. niger, A. oryzae, A. oryzae var. viridis, A. proliferans, A. sojae Aspergillus sp, A. terreus, A. terreus var. aureus, A. ustus, A. versicolor, A. wentii, Aureobasidium mausonii, A. pullulans Auricularia polytricha, Basidiobolus haptosporus, Beauveria bassiana, Benjaminella multispora, B. poitrasii, Botryodiplodia theobromae, Botryotrichum piluliferum, Botrytis allii, Cephaliophora irregularis, Cephalosporium sp., Chaetomella raphigera, Chaetomium globosum, Cladosporium herbarum, Cladosporium sp., Claviceps paspali, C. purpurea, Cokeromyces recurvatus, Coriolus versicolor, Cunninghamella blakesleeana, C. echinulata, C. elegans, C. sp., Curvularia brachyspora, C. cymbopogonis, C. fallax, C. lunata, Daedalea flavida, Datronia mollis, Dipodascus uninucleatus, Flammulina velutipes, Fusarium moniliforme, F. oxysporum, F. proliferatum, Fusarium sp., F. tricinctum, Ganoderma lucidum, Georichum candidum, Gibberella fujikuroi, G. saubinetti, Gliocladium roseum, Gongronella butleri, Helminthosporium sp., Humicola grisea, Hymenochaete rubigonosa, Laetiporus sulphureus, Lenzites striata, Lepiota rhacodes, Monilinia fructicola, Mucor hiemails, M. plumbeus, Mucor sp., Mycotypha africana, M microspora, Myrothecium roridum, M verrucaria, Neurospora crassa, N. sitophila, Paecilomyces sp., P. varioti, Pencillium ochrochloron, Pencillium sp., P. argillaceum, P. asperosporum, P. chrysogenum, P. citrinum, P. frequentans, P. funiculosum, P. janthinellum, P. lignorum, P. notatum, P. ochrochloron, P. pinophillum, P. purpurogenum, P. roqueforti, P. variabile, Phaenerochaete chrysosporium, Phialophora bubakii, P. calciformis, P. fastigiata, P. lagerbergii, P. richardsiae, Phialophora sp., Phoma exigua, Phycomyces blakesleeanus, Pleurotus flabellatus, P. florida, P. floridanus, P. ostreatus, P. sajor-caju, Polyporus meliae, Poria placenta, Ptychogaster sp., Pycnoporus cinnabarinus, P. sanguineus, Rhizopus oryzae, R. stolonifer, Saccharomyces cerevisiae, Sclerotium rolfsii, Scopulariopsis brevicaulis, Sporothecium sp., Sporotrichum sp., Stachybotrys chartarum, Stemphylium sarcinaeforme, Stemphylium sp., Tolypocladium inflatum, Trametes cubensis, T. hirsuta, T. lactinea, T. serialis, T. versicolor, T. inaequalis, T. harzianum, T. reesei, Trichoderma sp., T. viride, Trichosporon sp., Trichothecium roseum, Ustilago maydis, Volvariella diplasia, Volvariella sp. and V. volvacea.* Most preferred microorganisms for use in the method of the invention are *Acetobacter species, Bacillus coagulans, B. racemilacticus, B. laevolacticus, Corynebacterium glutamicum, Escherichia coli, Gluconobacter species, Pseudomonas species, lactic acid bacteria, Aspergillus niger, Aspergillus terreus* and *Saccharomyces cerevisiae*. A suitable inoculum density of microorganism(s) for use in the method of the invention is the density of microorganism(s), resulting in the lowest production costs of the fermentation product while providing good growth, metabolic and fermentation capabilities, with the selected type and concentration of biomass, the selected conditions of pretreatment (e.g., pH, time period and temperature) and the selected conditions for SSF (e.g., enzyme(s) and temperature). In a preferred embodiment the density of microorganism(s) is from about 0.1 to about 50, more preferably from about 2 to about 20, yet more preferably from about 5 to about 10 g of microorganism(s)/kg of pretreated lignocellulosic biomass.

Alternatively or in combination with a previous preferred embodiment, in a further preferred embodiment, the total solids concentration in the SSF range from about 5% to about 50%, such as from about 5% to about 40%, 30% or 25%, most preferably from about 40% to about 50%.

The optimum temperature for carrying out SSF, depends on the temperature optimum of the microorganism(s) and/or of the enzyme or enzyme mixtures. The person skilled in the art will know how to determine the optimum temperature for carrying out SSF. The optimum temperature to be used in combination with one or more microorganism(s) and/or enzyme(s) can be established by analysing the activity of the microorganism(s) and/or enzyme(s) under different temperature conditions using known methods. In a preferred embodiment, the temperature during SSF is within the range of about 20 to about 80°C, more preferably within the range of about 25 to about 60°C, and most preferably within the range of about 30 to about 50°C.

Alternatively or in combination with previous preferred embodiment, in a further preferred embodiment, the pH during SSF is adjusted and/or maintained to be between about 2.0 and 10.0, more preferably between about 4.0 and 8.0, more preferably between about 5.0 and 7.0 and most preferably the pH during SSF is adjusted and/or maintained to be at about 6.0. The pH may be controlled by (e.g. automatic) addition of alkaline pretreated biomass and optionally alkali in the form of a solution or suspension, for example by means of a pump or feeder whose output is set by a controller (computer) on basis of the desired pH value and the pH value as determined by a standard pH electrode.

Alternatively or in combination with previous preferred embodiment, in a further preferred embodiment the pH during SSF may be controlled by addition of the alkaline pretreated lignocellulosic biomass, without the need for an additional source of alkali and without large fluctuations in pH. In another preferred embodiment, the pH during SSF may be controlled by addition of the alkaline pretreated lignocellulosic biomass and an alkaline agent.

In a preferred embodiment, the SSF may comprise a pre-hydrolysis phase, a fed-batch phase with pH control by addition of alkaline pretreated lignocellulosic biomass and a batch phase with pH control by addition of an alkali. In a preferred embodiment, the fed-batch phase is accompanied and/or followed by a batch phase in which the alkali is added to counterbalance the pH decrease caused by the production of the organic acid, maintaining the pH at a constant level. In a preferred embodiment, the alkali is selected from the group consisting of, but not limited to: calcium hydroxide $(Ca(OH)_2)$, calcium oxide (CaO), ammonia $(NH_3)$, sodium hydroxide (NaOH), potassium hydroxide (KOH), sodium carbonate, urea and combinations thereof.

Alternatively or in combination with a previous preferred embodiment, in a further preferred embodiment, the fermentation product is produced in a quantity of at least 50%, more preferably at least 70%, even more preferably at least 90%, even more preferably at least 95%, even more preferably at least 98%, most preferably 100% of the theoretical maximum. The theoretical maximum can be calculated according to the following equation:

$$LA_{theor\,max} = DM * F * HF * FF$$

wherein

  $DM$ = total dry matter of alkaline pretreated lignocellulosic biomass (g);
  $F$ = fraction of polysaccharides (g) per gram of alkaline pretreated lignocellulosic biomass;
  $HF$ = hydrolysis factor to convert the molecular weight of the polysaccharides into the resulting monosaccharides;
  $FF$ = fermentation factor of 1.00 g of fermentation product per g of monosaccharides.

Step c) recovery

The term "recovery" is herein defined as any method or combination of methods in which the fermentation product of the invention is obtained from the SSF mixture of step (b) in a purer and/or more concentrated form, for example to obtain the fermentation product with a lower concentration of other components or a lower number of other components as compared to the SSF mixture of step (b).

In another aspect, the present invention relates to a reactor comprising a container for the alkaline pretreatment of lignocellulosic biomass optionally or temporarily linked to a fermentor for the simultaneous saccharification and fermentation (SSF) of the alkaline pretreated lignocellulosic biomass, wherein the reactor is for use in the method of the present invention, and wherein:

  1) the container comprises:

i) a mixing device;

ii) a heating device; and

iii) optionally, means for pre-extraction of soluble components from the lignocellulosic biomass;

2) the fermentor comprises:

i) automatic pH control; and

ii) an inlet for alkaline pretreated lignocellulosic biomass obtainable by claim 1 step a) from the container, which is controlled by the automatic pH control.

In a preferred embodiment, the mixing device is able to mix an alkaline agent into the alkaline pretreated lignocellulosic biomass.

In another preferred embodiment, the heating device is able to heat the mixture of an alkaline agent and the alkaline pretreated lignocellulosic biomass to the required process temperature. In a preferred embodiment, the mixture is heated by electrical heating or by steam.

In a preferred embodiment, the linking device or linking means between the container and the fermentor is a pump, preferably a screw feeder to allow automatic feeding of the alkaline pretreated lignocellulosic biomass into the fermentor. The linking device or linking means need not necessarily be present, or need not be physically linked, during the pre-treatment phase, but is preferably put in place, added or attached at least prior to and/or during the SSF phase. The linking device or linking means may thus be temporally or optionally present. In a different embodiment the linking device or linking means between the container and the fermentor is permanently present.

In a preferred embodiment, the alkali may be selected from the alkali that were described above.

In another preferred embodiment, the fermentor comprises one or more of the following: an inlet for automatic feeding of an alkali, which is controlled by the automatic pH control; an inlet for one or more enzyme(s) or enzyme mixture(s), for one or more microorganism(s) and/or an acid or base, e.g. sulphuric acid or $Ca(OH)_2$, more preferably 3M sulphuric acid or 20% w/v $Ca(OH)_2$; an outlet for sampling and/or monitor; automatic temperature control; and/or a stirrer assembly.

In another aspect, the invention relates to use of the reactor of the invention as described above, for the production of an organic acid from lignocellulosic biomass according to the method of the invention.

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one".

Description of the figured

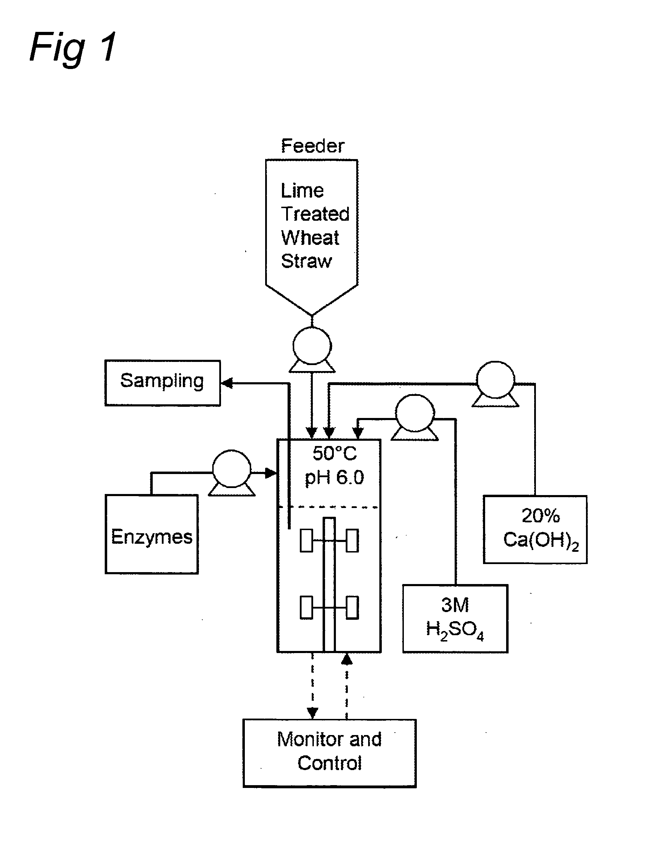

Figure 1. Schematic representation of the simultaneous saccharification and fermentation of lime-treated wheat straw to lactic acid.

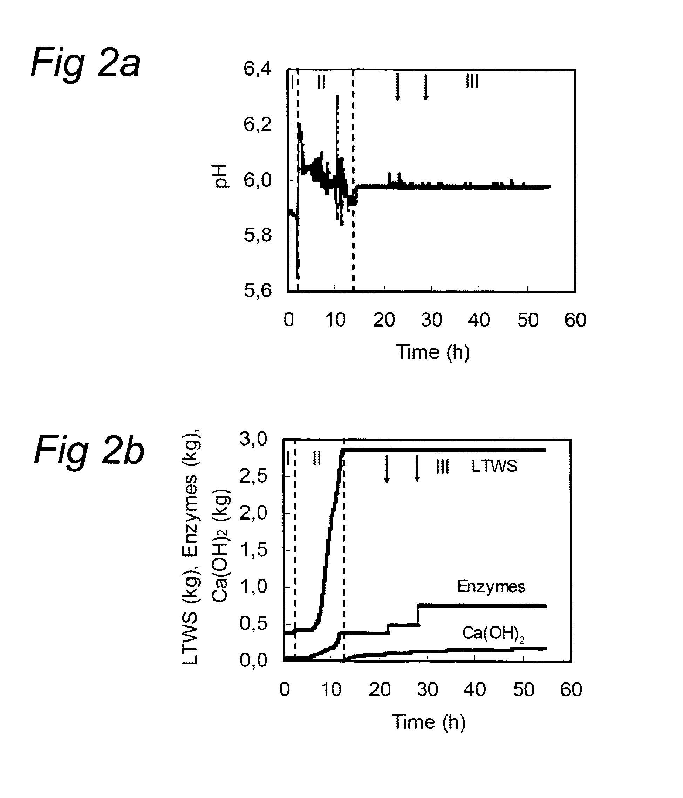

Figure 2. Control of pH (A) during simultaneous saccharification and fermentation of lime-treated wheat straw by commercial enzyme preparation GC 220 and *Bacillus coagulans* DSM 2314 *(B).* The areas between the dotted lines represent the pre-hydrolysis phase (I), the fed-batch phase (II) with pH control by addition of alkaline LTWS and enzymes, and the batch phase (III) with pH control by addition of $Ca(OH)_2$ suspension. Extra enzyme preparation GC220 was added at the times indicated by the arrows.

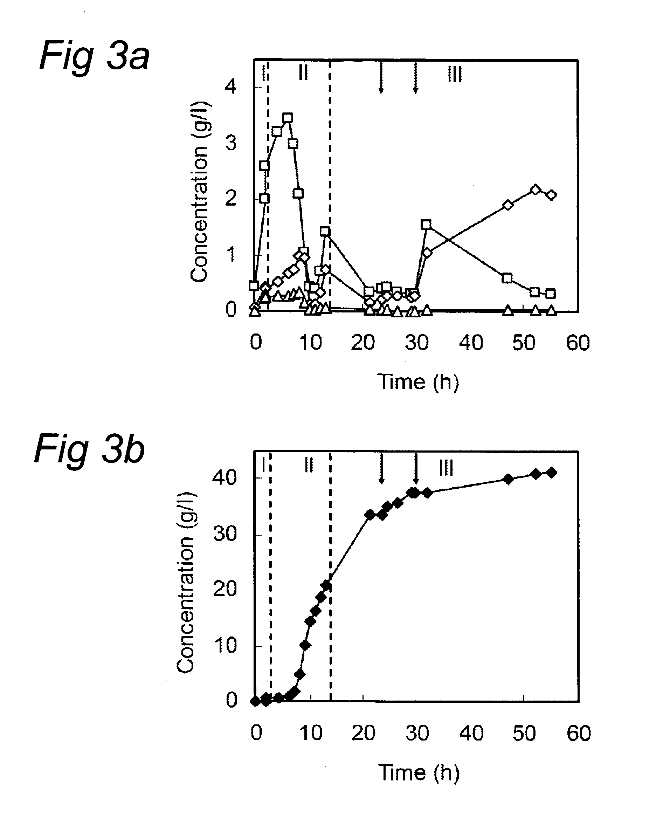

Figure 3. Profiles of glucose (□), xylose (◊), arabinose (△) *(A)* and lactic acid (♦) *(B)* in simultaneous saccharification and fermentation of lime-treated wheat straw by commercial enzyme preparation GC 220 and *Bacillus coagulans* DSM 2314. The areas between the dotted lines represent the pre-hydrolysis phase (I), the fed-batch phase (II) and the batch phase (III). Extra enzyme preparation GC220 was added at the times indicated by the arrows.

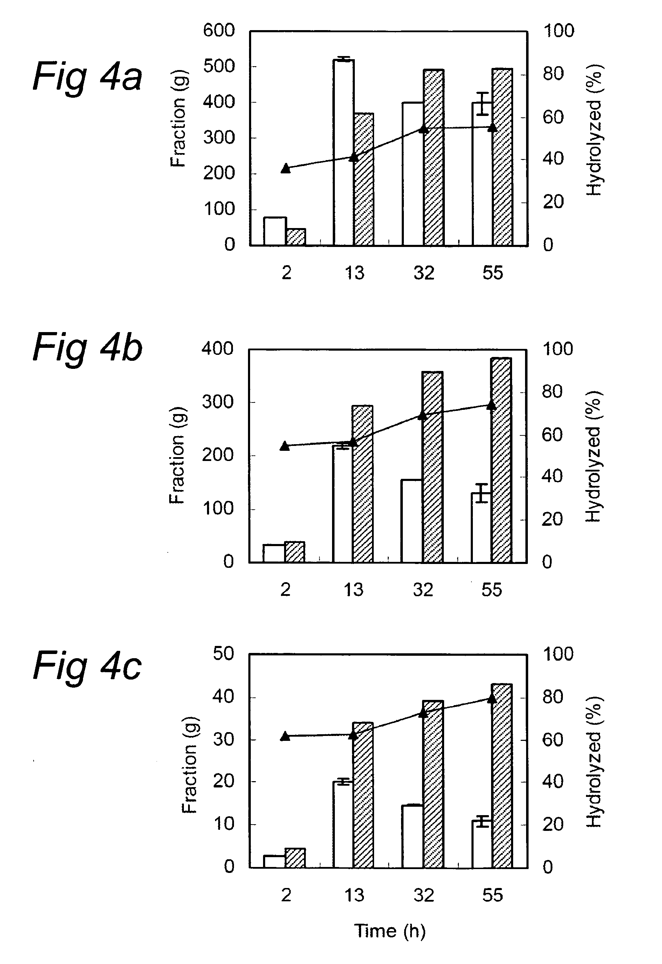

Figure 4. Insoluble fraction (□) and hydrolyzed soluble fraction (▨) (g) (calculated by the difference between initial amounts and analyzed insoluble amounts) of the polysaccharide glucan *(A),* xylan *(B)* and arabinan *(C)* at various time points during the simultaneous saccharification and fermentation of lime-treated wheat straw. Figure represents also the percentage of polysaccharide hydrolyzed into soluble products (▲). The error bars denote the deviation of duplicate analysis.

## Examples

## Example 1. Feedstock and pretreatment

Wheat straw was selected as lignocellulose model feedstock and was purchased from a farm in the Northeast of the Netherlands. The wheat straw was air dry (89.5% (*w/w*) dry matter) and ground through a 2-mm screen. The lime pretreatment was performed by filling two 15 l mixers (Terlet, The Netherlands), both with 1650 g ground wheat straw, 13 kg tap water and 165 g calcium hydroxide. This wheat straw suspension was heated and kept at 85°C for 16 hours under continuously stirring at 30 rpm. The lime-treated wheat straw (LTWS) suspension was subsequently cooled to 30°C, dehydrated by placing the LTWS in a cotton bag, and pressing the suspension using a manual piston press at pressure up to 9.7 kg/m$^2$. After dehydration, an amount of 11.45 kg LTWS with an average dry matter content of 27.0% (*w/w*) and pH 11.8 was obtained and served as substrate for further experiments. The chemical composition of LTWS was determined as described by van den Oever et al. (Van den Oever et al. (2003) J. Mater. Sci. 38:3697-3707).

## Example 2. Enzyme preparation

The enzyme preparation GC 220 (Genencor-Danisco, Rochester, USA) containing cellulase, cellobiase and xylanase activity of 116, 215 and 677 U/ml, respectively, (Kabel et al. (2006) Biotechnol. Bioeng. 93(1):56-63) and was used for this study. The preparation had a specific gravity of 1.2 g/ml and contained 4.5 mg/ml glucose, 2.9 mg/ml mannose and 0.8 mg/ml galactose.

## Example 3. Micro-organism and pre-culture

The bacterium *Bacillus coagulans* strain DSM 2314 (available at the DSMZ - Deutsche Sammlung von Mikroorganismen und Zellkulturen GmbH, Inhoffenstraße 7 B, 38124 Braunschweig, Germany) was used as lactic acid-producing micro-organism. Bacterial cells were maintained in a 10% (w/w) glycerol stock solution and stored at -80°C. Chemicals, unless indicated otherwise, were purchased from Merck (Darmstadt, Germany). Gelrite plates were prepared with medium containing (per liter) glucose, 10 g; Gelrite, 20 g (Duchefa, Haarlem, The Netherlands); yeast extract, 10 g (Duchefa); $(NH_4)_2HPO_4$, 2 g; $(NH_4)_2SO_4$, 3.5 g; BIS-TRIS, 10 g (USB, Ohio, USA); $MgCl_2.6H_2O$, 0.02 g and $CaCl_2.2H_2O$, 0.1 g. Glucose and Gelrite were dissolved in stock solution A (4 times concentrated). The pH of this stock solution was adjusted to 6.4 with 2M hydrochloric acid and autoclaved for 15 min at 125°C. The remaining nutrients were dissolved in stock solution B (1.33 times concentrated) which was also adjusted to pH 6.4 with 2M hydrochloric acid but was filter sterilized (cellulose acetate filter with pore size of 0.2 $\mu$m, Minisart, Sartorius). After sterilization, the medium was prepared by combining stock solution A and B and Gelrite plates were poured. The bacteria were cultivated on Gelrite plates for 48 h at 50°C.

An isolated colony was used to inoculate a 100-ml broth with similar composition and preparation as described above, however without the addition of Gelrite. The culture was incubated statically for 24 h at 50°C and functioned as inoculum for a 1400-ml broth. This culture was incubated also statically for 12 h at 50°C and served as a 10% (v/v) pre-culture for the SSF experiments.

## Example 4. Simultaneous saccharification and fermentation

The SSF of LTWS was carried out in a 20L-fermenter (Applikon, Schiedam, The Netherlands) with pH and temperature control (biocontroller ADI 1020). At the start of SSF, the fermenter was filled with 6.0 kg tap water and 1400 g dehydrated LTWS (DM content of 27.0% (w/w)). The following nutrients were then added to the LTWS suspension: yeast extract, 150 g (Duchefa); $(NH_4)_2HPO_4$, 30 g; $(NH_4)_2SO_4$, 52.5 g; $MgCl_2.6H_2O$, 0.3 g and $CaCl_2.2H_2O$, 1.5 g. The LTWS suspension was then heated to 50°C and the pH was adjusted to 6.0 with 101 g 3M sulphuric acid (- 30 g $H_2SO_4$). The SSF process of LTWS to lactic acid consisted of three phases; I) the pre-hydrolysis phase of pre-loaded LTWS, II) the fed-batch phase with automatic feeding of LTWS from a screw feeder and III) the batch phase with pH control by a calcium hydroxide suspension and no LTWS feeding. A schematic representation of the experimental setup is shown in Figure 1. The pre-hydrolysis was initiated by addition of 40 ml enzyme preparation (88 mg enzyme/g DM substrate) to the LTWS suspension and was incubated for two hours at 50°C under continuously stirring at 250 rpm. The fed-batch phase was initiated by addition of 1500-ml pre-culture of *B. coagulans* DSM 2314 to the fermenter. The lactic acid produced by the bacteria was neutralized by the automatic addition of 8623 g dehydrated LTWS (DM of 27.0%) to the fermenter through a feeder (K-Tron Soder Feeders, Canada) and was regulated by the pH of the medium which was set at 6.0. Throughout the fed-batch phase, an amount of 280 ml of enzyme preparation (total enzyme loading of 98 mg/g DM substrate) was added proportional to the LTWS addition rate into the fermenter. During the batch phase, the pH was controlled at 6.0 by the addition of 20.0% (*w/v*) calcium hydroxide suspension. Samples were withdrawn for dry matter, substrate and (by)-product analysis.

Example 5. Analytical methods

For the analysis of monomeric sugars, the fermentation broth samples were centrifuged (3 min at 17400×g), the pH of the supernatant was adjusted to 5.0 with barium carbonate using a pH-indicator (Bromophenolblue) followed by filtration of the liquid. The analysis was performed by high-performance anion-exchange chromatography using a Carbopack PA1 column (column temperature of 30°C) and a pulsed amperometric detector (ED50) (Dionex, Sunnyvale, CA). Prior to injection, the system was equilibrated with 25.5 mM NaOH for 10 min at a flow rate of 1.0 ml/min. For the separation of monomeric sugars, at injection the mobile phase was shifted to de-ionized water for 30 min. Post-column addition of sodium hydroxide was used for detection of the neutral monomeric sugars.

The determination of soluble oligomeric sugars was performed by centrifugation for 5 minutes at 3000 rpm (Centaur 2, Beun de Ronde, The Netherlands) of pre-weighed samples and freeze drying the supernatant overnight. Pellets were weighed, hydrolyzed with sulphuric acid and neutral monomeric sugars were determined according to the method as described by van den Oever et al. (Van den Oever et al. (2003) J. Mater. Sci. 38:3697-3707). For the calculations, an average molecular weight of oligomers from glucan and xylan of 166 and 132 g/mol, respectively, were applied, resulting in a hydrolysis factor of 1.08 and 1.14, respectively.

For the analysis of insoluble polymeric sugars, samples of 25 gram were centrifuged for 5 min at 3000 rpm (Centaur 2, Beun de Ronde, The Netherlands), supernatant was removed and the pellet was washed by re-suspension in 25 ml fresh demineralised water following by a centrifugation step of 5 minutes at 3000 rpm (Centaur 2, Beun de Ronde, The Netherlands). The sequence of re-suspension and centrifugation was repeated three times. After the last removal of the supernatant, the pellets were freeze dried overnight. The pellets were weighed (values used for dry matter (DM) calculation), polymeric material hydrolyzed with sulphuric acid and neutral sugars analyzed according to the method as described by van den Oever et al. (Van den Oever et al. (2003) J. Mater. Sci. 38:3697-3707). For the calculations, a molecular weight of glucan and xylan of 162 and 132 g/mol, respectively, were applied and resulting in a hydrolysis factor of polymer to monomer of 1.11 and 1.14, respectively.

The analysis of organic acids was performed by high pressure liquid chromatography according to the procedure described by Maas et al. (Maas et al. (2006) Appl. Microbiol. Biotechnol. 72:861-868).

The chiral purity (%) of lactic acid was determined by derivatization of all lactates using methanol, after which both enantiomers of methyl lactate were separated on a chiral Gas Chromatography column and detected using a Flame Ionization Detector. The chiral purity was expressed as the area of the main enantiomer divided by the sum of areas of both enantiomers.

Example 6. Calculations

The theoretical maximum lactic acid $(LA_{theor.max.}$ (g)) production was calculated according the following equation [Eq. 1].

$$LA_{theor.max.} = DM_{substrate} * F_{polysacch.} * HF_{monosacch./polysacch.} * FF \qquad [\text{Eq. 1}]$$

Where $DM_{substrate}$ = the total Dry Matter of substrate LTWS (g); $F_{polysacch.}$ = Fraction polysaccharides per substrate (g/g); $HF_{monsacch.\cdot lpolysacch.}$ = Hydrolysis Factor of polysaccharides, incorporation of water results in 1.11 g hexose from 1.00 g glucan and 1.14 g pentose from xylan and arabinan (g/g) and $FF$ = Fermentation Factor of 1.00 g lactic acid per g of monomeric sugar.

The efficiency of the enzymatic hydrolysis (%, *w/w*) was based on the amount of hydrolyzed polysaccharides (g) (calculated by the difference between initial amounts and analyzed insoluble amounts) divided by the amount of polysaccharides (g) initially present in the substrate. The fermentation efficiency (%, *w/w*) is expressed as the amount of lactic acid produced (g) divided by the amount of monomeric sugars consumed (g) by the bacteria. The overall efficiency of the SSF (%, *w/w*) was calculated by the amount of lactic acid produced (g) divided by the theoretical maximum amount of lactic acid (g) determined as described in Eq. 1.

Example 7. Simultaneous saccharification and fermentation of LTWS to lactic acid

The polysaccharide composition of the lime-treated wheat straw (LTWS) consisted mainly of glucan, xylan and arabinan of 33.0, 19.0 and 2.0% (*w/w*), respectively, whereas the remaining mass constituted of lignin, ash, extractives and uronic acids. Some of the soluble components in wheat straw were partially removed by the solid/liquid separation (dehydration) of the LTWS. The focus of this study was on the conversion of glucan, xylan and arabinan which are the predominant polysaccharides present in LTWS and accounted for 99.8% (*w/w*) of the total polymeric sugars. Previous work showed that the cellulase preparation GC 220, used for the saccharification of polysaccharides, functioned optimally at 50°C and pH 5.0 (manuscript in submission), whereas growth conditions for *Bacillus coagulans* DSM 2314 were 54°C and pH 6.5 (WO 2004/063382). In this study, both the enzymatic hydrolysis and the fermentation occurred simultaneously in the same reactor at compromising conditions which were set at 50°C and pH 6.0.

The SSF of LTWS to lactic acid was studied in a 20L controlled stirred fermenter. Previous results showed that when this process was performed without a pre-hydrolysis of an initial amount of LTWS, the concentration of monomeric sugars was low and resulted, therefore, in relatively low lactic acid productivity. As a consequence, the fed-batch addition rate of the alkaline substrate to neutralize the produced lactic acid was low (results not shown). In order to start the fermentation with a substantial initial amount of fermentable sugars (> 2 g/l), a pre-hydrolysis of 378 g LTWS and enzyme preparation (88 mg per g DM LTWS) in approximately 6 liter volume at pH 6.0 for two hours was introduced. This resulted in glucose, xylose and arabinose concentrations of 2.0, 0.4 and 0.3 g/l, respectively (Fig. 3A).

The second phase (II) was initiated by introducing a 1500-ml pre-culture of *B. coagulans* DSM 2314. A minor amount of lactic acid produced in the pre-culture caused a slight pH decrease and was automatically neutralized by the addition of LTWS (Fig. 2A, B). After a lag phase of four hours, the dissolved oxygen concentration decreased rapidly from 100% to oxygen-limiting conditions of below 1% (results not shown). At that moment, a concentration of glucose, xylose and arabinose of 3.3, 0.7 and 0.3 g/l, respectively, was present (Fig. 3A). These sugars were consumed simultaneously where glucose was utilized faster than xylose and arabinose.

Simultaneous with the consumption of these monomeric sugars, lactic acid was produced which was neutralized by the automatic addition of alkaline LTWS. By the addition of alkaline substrate throughout the fed-batch phase, the pH was maintained accurately at 6.0 ± 0.1 (Fig. 2A, B). At the end of phase II, a total amount of 10023 g dehydrated LTWS (- 2706 g DM LTWS) and 320 ml of enzyme preparation was added to the fermenter. A lactic acid concentration of 20.5 g/l supernatant was detected (Fig. 3B), corresponding to a total of 342 g lactic acid. The chiral L(+) purity of lactic acid was determined at 99.4% which is similar to that obtained with xylose as sole carbon source (WO 2004/063382).

At the end of phase I, a low acetic acid concentration was detected in the medium which increased to 1.5 g/l throughout phase II but, remained constant during phase III (results not shown). This indicates that acetic acid was most likely not a fermentation product formed by *B. coagulans.* Acetic acid can be released upon solubilisation and hydrolysis of hemicellulose during chemical pretreatment (Palmqvist et al. (1999) Biotechnol. Bioeng. 63(1): 46-55). By the dehydration procedure of the LTWS, part of the acetic acid was easily separated from the substrate by removing the press water. Apparently, a remaining amount of acetic acid was fed together with the substrate to the fermenter. Also, minor traces of other organic acids such as succinic acid and formic acid (<0.5 g/l) were detected in the fermentation broth.

Phase III was initiated by changing the pH control from the addition of alkaline LTWS to a 20% (*w/v*) calcium hydroxide suspension. To maintain the pH at 6.0, addition of calcium hydroxide suspension occurred relatively fast but shifted, however, after a few hours to a lower addition rate indicating a decline of the volumetric lactic acid productivity (Fig. 2B, 3B). To exclude limitation (e.g. by inactivation) of enzymes, an extra dosage of enzyme preparation (80 ml) was added to the fermenter after 23.5 h of incubation. This resulted immediately in a slight acceleration of the calcium hydroxide addition rate indicating an increased lactic acid productivity and limitation of enzymatic activity (Fig. 3B). Nevertheless, after 29.7 h of incubation, a decline of the calcium hydroxide addition rate was observed again. Therefore, a second extra dosage of the enzyme preparation (240 ml) was added and resulted this time in a slight accumulation of glucose and xylose of 1.5 and 1.0 g/l (Fig. 3A), respectively, indicating that microbial conversion instead of enzymatic hydrolysis was rate limiting. After 32 h of incubation, a lactic acid concentration of 37.1 g/l was obtained, with a chiral L (+)-lactic acid purity of 99.4%. Continuation of the SSF process to a total incubation period of 55 h resulted in a slightly increased lactic acid concentration of 40.7 g/l supernatant (-37.8 g lactic acid/kg fermentation broth) with an overall volumetric lactic acid productivity of 0.74 g/l/h. At this stage, a chiral L(+)-lactic acid purity of 97.2% was analyzed. This slight decline in lactic acid purity is possibly a result of infection with other undesired lactic acid-producing microorganisms. Since the substrate used was not sterile and also the chemical pretreatment and fermentation occurs in an open system under non-sterile conditions, microbial contamination throughout the SSF process is possible.

Example 8. Conversion efficiency

The efficiency of the enzymatic hydrolysis of the polymeric material present in LTWS is shown in Figure 4. The insoluble polymeric fraction was determined at various time points throughout the SSF experiment. At the end of the pre-hydrolysis (2 h) of 378 g LTWS, 36% of the insoluble glucan (Fig. 4A), 55% of xylan (Fig. 4B) and 62% of arabinan (Fig. 4C) was converted to soluble saccharides including monomeric sugars and oligomeric sugars. After the fed-batch phase (13 h), 2706 g LTWS was added and resulted in a conversion of 42% of glucan, 57% of xylan and 63% of arabinan to products including soluble saccharides and lactic acid. Between 13 and 32 h of incubation, further hydrolysis of the polymeric sugars was observed. However, during the last 23 h of the SSF, minor hydrolysis of the polysaccharides occurred and this corresponded with the decline in lactic acid productivity during this phase. After 55 h, 398 g of glucan, 130 g of xylan and 11 g of arabinan was still present as insoluble polymeric material. With these values, the hydrolysis efficiency of the initial glucan, xylan and arabinan present in LTWS were calculated as 55, 75 and 80%, respectively. The monomeric sugars, derived from the LTWS, were partly converted to lactic acid (711 g) by *B. coagulans* and accounted for 81% (*w/w*) of the theoretical maximum, indicating the formation of other products such as microbial biomass and carbon dioxide. An overall conversion yield of 43% (*w/w*) of the theoretical maximum was calculated according to Equation 1. The fate of polysaccharides initially present in LTWS after 55 h of incubation is shown in Table I. A part of the polysaccharides present in LTWS, remained as insoluble polysaccharides (37% *w/w*) whereas a minor part was converted into soluble oligomeric (5% *w/w*) and monomeric (3% *w/w*) sugars. Another part of the initial polysaccharides present in the LTWS was not recovered in the form of saccharides or lactic acid and was therefore ascribed as 'unaccounted'.

Table I. Fate of polysaccharides[a] initially present in lime-treated wheat straw after 55 h of simultaneous saccharification and fermentation. Presented values are averages based on duplicate analytical measurements.

| Fraction | Percentage (% *w/w*) |
| --- | --- |
| Polysaccharides (insoluble)[b] | 37 |
| Oligosaccharides (soluble) | 5 |
| Monosaccharides (soluble) | 3 |
| Lactic acid (soluble) | 43 |
| Unaccounted (insoluble/soluble)[c] | 13 |

[a]Total of glucan, xylan and arabinan
[b]Part of the initial polysaccharides remained present as insoluble polysaccharides
[c] Part of the initial polysaccharides was not recovered and therefore denoted as 'unaccounted'

Example 9. Neutralization of acid by alkaline substrate

The lactic acid produced (342 g) during the fed-batch phase (II) was neutralized with alkaline pretreated wheat straw. During this phase, an amount of 2328 g LTWS was added to the fermenter. Together with this substrate, an amount of 230 g calcium hydroxide was added to the fermenter and accounted for a ratio of 0.67 g calcium hydroxide per g of lactic acid. The lactic acid (369 g) produced during the batch phase (III) was neutralized with 163 g calcium hydroxide resulting in a ratio of 0.44 g lactic acid per g calcium hydroxide.

Discussion

Lignocellulosic feedstocks are considered as potential attractive substrates for the production of bulk chemicals. Pretreatment of biomass is required in order to break open the lignocellulosic matrix, an enzymatic hydrolysis is necessary for the hydrolysis of polymeric carbohydrates. The lime pretreatment has proven to enhance enzymatic digestibility of the polysaccharides present in lignocelluloses (Chang et al (1998) Appl. Biochem. Biotechnol. 74:135-159; Kaar and Holtzapple (2000) Biomass and Bioenergy 18:189-199) and results, in comparison to other pretreatment routes, in minor inhibitor formation. However, prior to the enzymatic hydrolysis, it is essential to adjust the pH to a level optimal for enzymatic activity. In this study, the reduction of pH by washing or neutralization was omitted by using the alkaline character of LTWS in order to neutralize lactic acid produced by microbial fermentation during a SSF process.
The results showed that the largest part of the polysaccharides in LTWS was converted enzymatically and the resulting sugars were fermented simultaneously to mainly lactic acid by *B. coagulans* DSM 2314. Between 10 and 30 h of incubation, the bacteria utilized the monomeric sugars, as soon as they appeared in the medium, resulting in relatively low monomeric sugar concentrations (< 2 g/l). This indicates that throughout this period, the enzymatic hydrolysis was the rate-controlling step. The highest lactic acid productivity was observed during the fed-batch phase and the initial hours of the batch phase and declined rapidly after approximately 20 hours of incubation, as shown in Figure 3B. An extra addition of enzyme preparation showed a slight improvement of the volumetric lactic acid productivity but shifted within a few hours again to a relatively low production rate. A second extra enzyme addition did not affect the lactic acid productivity significantly (Fig. 3B). This addition of new enzymes resulted in a modest liberation of hemicellulose sugars (xylose, arabinose) but no further hydrolysis of glucan occurred. This shows that the remaining glucan was too recalcitrant or not accessible for further hydrolysis, resulting in decreasing lactic acid productivity. Another possible explanation of the decreased lactic acid productivity is the inhibition of enzymes and/or bacteria by the increasing lactic acid concentration.

A lactic acid concentration of 40.7 g/l supernatant (~37.8 g lactic acid/kg fermentation broth) with a relatively high chiral purity was determined after 55 hours of incubation, corresponding to an overall lactic acid yield of 43% of the theoretical maximum. Moreover, the efficiencies of the enzymatic saccharification and the fermentation were both determined. These calculations showed that, based on residue analysis, at the end of the SSF process (55 h) 55% of the glucan, 75% of the xylan and 80% of the arabinan present in LTWS was enzymatically hydrolyzed which agree well with previously obtained results. In order to improve the yield it is necessary to decrease the recalcitrance or improve the accessibility of polymeric sugars in the LTWS by optimization of the pretreatment procedure. The concentrations of soluble monosaccharides and oligosaccharides in the medium were relatively low which can be expected in a SSF process. A fermentation yield of 81% was determined and is slightly better than the results obtained by Otto (*supra*) who reported the production of 35 g/l lactic acid from 50 g/l xylose as sole carbon source. Since no other soluble fermentation products were detected, the remaining 19% of the LTWS derived monomeric sugars were most presumably converted to bacterial biomass and some carbon dioxide during the aerobic part of the fermentation.

During the fed-batch phase (II) it was possible to counterbalance the pH decrease caused by lactic acid production by addition of the alkaline feedstock, showing that lime treatment can be combined well with the production of a wide range organic acids from lignocellulosic biomass. Throughout this phase, the ratio of calcium hydroxide in LTWS added per produced lactic acid was determined at 0.67 g/g. The theoretical stoichiometric neutralization of 1.00 g lactic acid requires 0.41 g calcium hydroxide. Therefore, only 61% of the calcium hydroxide initially added to the wheat straw was used for lactic acid neutralization. On the other hand, throughout the batch phase (III), an alkaline/acid ratio of 0.44 g/g was calculated corresponding to 93% of the added calcium hydroxide suspension used for lactic acid neutralization. The low efficiency of the calcium hydroxide added with the LTWS for lactic acid neutralization during phase II has three possible explanations. Firstly, part of the calcium hydroxide could have been used during the chemical pre-treatment of the wheat straw such as the neutralization of acetic acid or other organic acids and/or irreversible binding to the lignin. Secondly, the calcium hydroxide might be released slowly from the insoluble wheat straw fibers and could therefore partly have been used for lactic acid neutralization in the fed-batch phase. Finally, besides lactic acid production, other acidification reactions could have contributed to the decrease of pH and therefore the demand of alkaline substrate. For instance the uptake and dissociation of the nitrogen source ammonium by the micro-organism into ammonia and protons (Guebel (1992) Biotechnol. Lett. 14(12):1193-1198).

The results in this paper show that it is possible to use lignocellulosic materials for the production of lactic acid. Lignocellulosic biomass is a relatively inexpensive substrate and this affects feedstock costs for lactic acid production positively. Nevertheless, in comparison to the traditional relatively 'clean' feedstocks with well defined composition, using heterogenic lignocellulosic substrates will require a more intensified down stream processing (DSP) to recover and purify the lactic acid from the complex fermentation broth. The costs of feedstock materials and operational costs of the DSP contribute considerably to the overall production costs of lactic acid (Åkerberg and Zacchi (2000) Bioresour. Technol. 75:119-126). Whether the cost decrease of using lignocellulosic feedstocks outweighs the potential increasing costs of DSP was not analyzed at the moment.

In summary, lime-treated wheat straw was converted into L(+)-lactic acid by *B. coagulans* throughout a simultaneous saccharification and fermentation process at 20L bench-scale. The pentose and hexose sugars derived from the polymeric material were utilized simultaneously by *B. coagulans* resulting in a final lactic acid concentration of 40.7 g/l supernatant which accounted for 43% (w/w) of the theoretical yield. To our knowledge, this is the first evidence that a process having a combined alkaline pretreatment of lignocellulosic biomass and pH control in organic acid fermentation results in a significant saving of lime consumption and avoiding the necessity to recycle lime.

## Claims

1. A method for the production of an organic acid as a fermentation product from lignocellulosic biomass, comprising the steps of:

   a) pretreatment of lignocellulosic biomass with an alkaline agent to obtain alkaline pretreated lignocellulosic biomass with a pH of between about 8.0 and about 14.0 ;
   b) simultaneous saccharification and fermentation (SSF) of the alkaline pretreated lignocellulosic biomass of step a) in a fermentor, whereby the decrease in pH, caused by the production of the organic acid, is counteracted by the addition of alkaline pretreated lignocellulosic biomass as obtained in step a) with a pH of between about 8.0 and about 14.0, optionally in combination with an alkali, to adapt the pH below about 8.0 and/or to maintain the pH at a specific pH below 8.0, allowing optimal activity of the micro-organism(s) and/or enzymes added; and
   c) optionally recovery of the fermentation product;

wherein the SSF of step b) comprises a pre-hydrolysis phase wherein the pH of a part of the alkaline pretreated biomass obtained by step a) is adapted by addition of one or more acids to between 2.0 and 8.0 to allow for conversion of alkaline pretreated biomass into fermentable sugars to start the microbial conversion of biomass into organic acids.

2.  A method according to claim 1, wherein the SSF of step b) further comprises the steps of:

   i) enzymatic hydrolysis with an hydrolytic enzyme to obtain fermentable saccharides; and
   ii) microbial fermentation using a microorganism which is able to convert the saccharides of step i) into the fermentation product.

3.  A method according to claim 1 or claim 2, wherein the SSF of step b) is operated in a chemostat mode, in which the alkaline pretreated lignocellulosic biomass is used as a nutrient.

4.  A method according to any one of the preceding claims, wherein the alkaline pretreated lignocellulosic biomass is added to the SSF of step b) in a fed-batch manner.

5.  A method according to any one of the preceding claims, wherein the pretreatment of lignocellulosic biomass is preceded by or combined and/or integrated with a mechanical comminution of lignocellulosic biomass, wherein the mechanical comminution preferably is selected from the group consisting of: milling, mechanical refining and extrusion.

6.  A method according to any one of the preceding claims, wherein the alkaline pretreated lignocellulosic biomass is subjected to one or more of the following steps prior to SSF:

   i) a cooling step;
   ii) a washing step; and/or
   iii) a dewatering step;

   wherein dewatering is preferably performed by using filtration while applying pressure to the pretreated biomass, wherein the applied pressure is ranged between 0 and about 100 bar.

7.  A method according to any one of the preceding claims, wherein the temperature during SSF is ranged between about 20 to about 80°C and/or wherein the pH during SSF is controlled between approximately 2.0 and approximately 10.0.

8.  A method according to any one of the preceding claims, wherein the pH during SSF is controlled by addition of the alkaline pretreated lignocellulosic biomass and an alkali and wherein the SSF preferably comprises a pre-hydrolysis phase, a fed-batch phase with pH control by addition of alkaline pretreated lignocellulosic biomass and a batch phase with pH control by addition of an alkali.

9.  A method according to any one of the preceding claims, wherein the lignocellulosic biomass is selected from the group consisting of: grass, wood, bagasse, straw, paper, plant material, and combinations thereof.

10. A method according to any one of the preceding claims, wherein the alkaline agent used in step a) is selected from the group consisting of: calcium hydroxide (Ca(OH)$_2$), calcium oxide (CaO), ammonia (NH$_3$), sodium hydroxide (NaOH), sodium carbonate, potassium hydroxide (KOH), urea and combinations thereof.

11. A method according to any one of claims 2 to 10, wherein the hydrolytic enzyme of step b) is selected from the group consisting of: cellulase preparations, hemicellulase preparations, cellobiase, xylanase preparations, amylase and pectinase.

12. A method according to any one of the preceding claims, wherein the organic acid is selected from the group consisting of: lactic acid, citric acid, itaconic acid, succinic acid, fumaric acid, glycolic acid, pyruvic acid, acetic acid, glutamic acid, malic acid, maleic acid, propionic acid, butyric acid, gluconic acid and combinations thereof.

13. A method according to any one of the preceding claims, wherein the microorganism is a bacterium, a fungus, an archea or an algae, wherein the microorganism is preferably selected from *Acetobacter species, Bacillus coagulans, B. racemilacticus, B. laevolacticus, Corynebacterium glutamicum, Escherichia coli, Gluconobacter species, Pseu-*

*domonas species, lactic acid bacteria, Rhizopus oryzae, Aspergillus niger, Aspergillus terreus* and *Saccharomyces cerevisiae*.

14. A reactor comprising a container for the alkaline pretreatment of lignocellulosic biomass linked to a fermentor for the simultaneous saccharification and fermentation (SSF) of the alkaline pretreated lignocellulosic biomass, wherein the reactor is for use in the method in any one of claims 1 to 13, and wherein:

   1) the container comprises:

      i) a mixing device;
      ii) a heating device; and
      iii) optionally, means for pre-extraction of soluble components from the lignocellulosic biomass;

   2) the fermentor comprises:

      i) automatic pH control; and
      ii) an inlet for alkaline pretreated lignocellulosic biomass obtainable by claim 1 step a) from the container, which is controlled by the automatic pH control,

   wherein the linking means between the container and the fermentor preferably is a pump, more preferably a screw feeder to allow automatic feeding of the alkaline pretreated lignocellulosic biomass into the fermentor; wherein the fermentor preferably comprises one or more of the following:

      a) an inlet for automatic feeding of an alkaline agent, which is controlled by the automatic pH control;
      b) an inlet for an enzyme, a microorganism and/or an acid or base;
      c) an outlet for sampling and/or monitor; and/or
      d) automatic temperature control
      e) a stirrer assembly.

15. Use of the reactor according to claim 14 for the production of an organic acid from lignocellulosic biomass according to the method of claims 1 - 13.

## Patentansprüche

1. Verfahren zur Herstellung einer organischen Säure als ein Fermentationsprodukt aus lignocelluloser Biomasse, welches die Schritte umfasst:

   a) Vorbehandlung lignocelluloser Biomasse mit einem alkalischen Stoff, um alkalisch vorbehandelte lignocellulose Biomasse mit einem pH zwischen etwa 8.0 und etwa 14.0 zu erhalten;
   b) gleichzeitige Saccharifizierung und Fermentation (SSF) der alkalisch vorbehandelten lignocellulosen Biomasse aus Schritt a) in einem Fermenter, wobei der Verringerung in einem pH, welche durch die Herstellung der organischen Säure verursacht wird, durch die Hinzufügung von alkalisch vorbehandelter lignocelluloser Biomasse wie in Schritt a) mit einem pH zwischen etwa 8.0 und etwa 14.0 erhalten, entgegengewirkt wird, optional in Kombination mit einem Alkali, um den pH unter etwa 8.0 anzugleichen und/oder um den pH bei einem bestimmten pH unter 8.0 aufrechtzuerhalten, wodurch eine optimale Aktivität des/der hinzugefügten Mikroorganismus/-men und/oder Enzymen ermöglicht wird; und
   c) optionale Gewinnung des Fermentationsproduktes;

   wobei die SSF aus Schritt b) eine Vor-Hydrolysephase umfasst, wobei der pH eines Teils der alkalisch vorbehandelten Biomasse, welche durch Schritt a) erhalten wird, durch Hinzufügung einer oder mehrerer Säuren auf zwischen 2.0 und 8.0 angeglichen wird, um eine Umsetzung alkalisch vorbehandelter Biomasse in fermentierbare Zucker zuzulassen, um die mikrobielle Umsetzung von Biomasse in organische Säuren zu beginnen.

2. Verfahren nach Anspruch 1, wobei die SSF aus Schritt b) weiter die Schritte umfasst einer:

   i) enzymatischen Hydrolyse mit einem hydrolytischen Enzym, um fermentierbare Saccharide zu erhalten; und
   ii) mikrobiellen Fermentation unter Verwendung eines Mikroorganismus, welcher die Saccharide aus Schritt i) in das Fermentationsprodukt umsetzen kann.

3. Verfahren nach Anspruch 1 oder Anspruch 2, wobei die SSF aus Schritt b) in einem Chemostat-Modus betrieben wird, in welchem die alkalisch vorbehandelte lignocellulose Biomasse als ein Nährstoff verwendet wird.

4. Verfahren nach einem der vorstehenden Ansprüche, wobei die alkalisch vorbehandelte lignocellulose Biomasse zu der SSF aus Schritt b) in einer Zuführ-StapelWeise hinzugefügt wird.

5. Verfahren nach einem der vorstehenden Ansprüche, wobei der Vorbehandlung lignocelluloser Biomasse eine mechanischen Zerkleinerung lignocelluloser Biomasse vorangeht oder damit kombiniert und/oder integriert wird, wobei die mechanische Zerkleinerung bevorzugt aus der Gruppe ausgewählt ist, die umfasst: Mahlen, mechanisches Raffinieren und Extrudieren.

6. Verfahren nach einem der vorstehenden Ansprüche, wobei die alkalisch vorbehandelte lignocellulose Biomasse einem oder mehreren der folgenden Schritte vor einer SSF unterworfen wird:

   i) einem Kühlschritt;
   ii) einem Waschschritt; und/oder
   iii) einem Entwässerungsschritt;

wobei eine Entwässerung bevorzugt unter Verwendung einer Filtration durchgeführt wird, während ein Druck auf die vorbehandelte Biomasse ausgeübt wird, wobei der ausgeübte Druck zwischen 0 und etwa 100 bar liegt.

7. Verfahren nach einem der vorstehenden Ansprüche, wobei die Temperatur während einer SSF zwischen etwa 20 bis etwa 80°C liegt und/oder wobei der pH während einer SSF zwischen näherungsweise 2.0 und näherungsweise 10.0 gesteuert wird.

8. Verfahren nach einem der vorstehenden Ansprüche, wobei der pH während einer SSF durch Hinzufügung der alkalisch vorbehandelten lignocellulosen Biomasse und eines Alkalis gesteuert wird und wobei die SSF bevorzugt eine Vor-Hydrolysephase, eine Zuführ-Stapel-Phase mit einer pH-Steuerung durch Hinzufügung alkalisch vorbehandelter lignocelluloser Biomasse und eine Stapelphase mit pH-Steuerung durch Hinzufügung eines Alkalis umfasst.

9. Verfahren nach einem der vorstehenden Ansprüche, wobei die lignocellulose Biomasse ausgewählt ist aus der Gruppe, die umfasst: Gras, Holz, Bagasse, Stroh, Papier, Pflanzenmaterial und deren Kombinationen.

10. Verfahren nach einem der vorstehenden Ansprüche, wobei der alkalische Stoff, welcher in Schritt a) verwendet wird, ausgewählt ist aus der Gruppe, die umfasst: Calciumhydroxid ($Ca(OH)_2$), Calciumoxid (CaO), Ammoniak ($NH_3$), Natriumhydroxid (NaOH), Natriumkarbonat, Kaliumhydroxid (KOH), Harnstoff und deren Kombinationen.

11. Verfahren nach einem der Ansprüche 2 bis 10, wobei das hydrolytische Enzym aus Schritt b) ausgewählt ist aus der Gruppe, die umfasst: Cellulase-Präparate, Hemicellulase-Präparate, Cellobiase, Xylanase-Präparate, Amylase und Pektinase.

12. Verfahren nach einem der vorstehenden Ansprüche, wobei die organische Säure ausgewählt ist aus der Gruppe, die umfasst: Milchsäure, Zitronensäure, Itakonsäure, Bernsteinsäure, Fumarsäure, Glykolsäure, Brenztraubensäure, Essigsäure, Glutaminsäure, Apfelsäure, Maleinsäure, Propionsäure, Buttersäure, Glukonsäure und deren Kombinationen.

13. Verfahren nach einem der vorstehenden Ansprüche, wobei der Mikroorganismus ein Bakterium, ein Fungus, ein Archaeen oder eine Alge ist, wobei der Mikroorganismus bevorzugt ausgewählt ist aus *Acetobacter Spezies, Bacillus coagulans, B. racemilacticus, B. laevolacticus, Corynebacterium glutamicum, Escherichia coli, Gluconobacter Spezies, Pseudomonas Spezies, Milchsäurebakterien, Rhizopus oryzae, Aspergillus niger, Aspergillus terreus* und *Saccharomyces cerevisiae.*

14. Reaktor, welcher einen Behälter für die alkalische Vorbehandlung von lignocelluloser Biomasse umfasst, welcher mit einem Fermenter für die gleichzeitige Saccharifizierung und Fermentation (SSF) der alkalisch vorbehandelten lignocellulosen Biomasse verbunden ist, wobei der Reaktor zur Verwendung in dem Verfahren in einem der Ansprüche 1 bis 13 vorgesehen ist, und wobei:

1) der Behälter umfasst:

i) ein Mischgerät;
ii) ein Heizgerät; und
iii) optional, eine Einrichtung zur Vor-Extraktion lösbarer Teile aus der lignocellulosen Biomasse;

2) der Fermenter umfasst:

i) eine automatische pH-Steuerung; und
ii) einen Einlass für alkalisch vorbehandelte lignocellulose Biomasse, welche durch Anspruch 1 Schritt a) aus dem Behälter erhaltbar ist, welcher durch die automatische pH-Steuerung gesteuert wird,

wobei die Verbindungseinrichtung zwischen dem Behälter und dem Fermentor bevorzugt eine Pumpe ist, hochbevorzugt ein Schneckenzuführer, um ein automatisches Hinzufügen der alkalisch vorbehandelten lignocellulosen Biomasse in den Fermenter zu erlauben; wobei der Fermenter bevorzugt eines oder mehreres des Folgenden umfasst:

a) einen Einlass zum automatischen Zuführen eines alkalischen Stoffes, welcher durch die automatische pH-Steuerung gesteuert wird;
b) einen Einlass für ein Enzym, einen Mikroorganismus und/oder eine Säure oder Base;
c) einen Auslass zum Probenziehen und/oder Überprüfen; und/oder
d) eine automatische Temperatursteuerung
e) eine Rühranordnung.

15. Verwendung des Reaktors nach Anspruch 14 zur Herstellung einer organischen Säure aus lignocelluloser Biomasse nach dem Verfahren der Ansprüche 1 bis 13.

**Revendications**

1. Procédé pour la production d'un acide organique sous forme d'un produit de fermentation à partir d'une biomasse lignocellulosique, comprenant les étapes de :

a) prétraitement d'une biomasse lignocellulosique avec un agent alcalin pour obtenir une biomasse lignocellulosique prétraitée avec un agent alcalin ayant un pH entre environ 8,0 et environ 14,0 ;
b) saccharification et fermentation simultanées (SFS) de la biomasse lignocellulosique prétraitée avec un agent alcalin de l'étape a) dans un fermenteur, où la baisse de pH, provoquée par la production de l'acide organique, est contrecarrée par l'addition d'une biomasse lignocellulosique prétraitée avec un agent alcalin tel qu'elle est obtenue dans l'étape a) ayant un pH entre environ 8,0 et environ 14,0, éventuellement en combinaison avec un alcali, pour adapter le pH en dessous d'environ 8,0 et/ou pour maintenir le pH à un pH spécifique en dessous de 8,0, ce qui permet une activité optimale du ou des micro- organismes et/ou enzymes ajoutés ; et
c) éventuellement récupération du produit de fermentation ; où la SFS de l'étape b) comprend une phase de pré-hydrolyse où le pH d'une partie de la biomasse prétraitée avec un agent alcalin obtenue par l'étape a) est adapté par l'addition d'un ou plusieurs acides à entre 2,0 et 8,0 pour permettre la conversion de la biomasse prétraitée avec un agent alcalin en sucres fermentables pour initier la conversion microbienne de la biomasse en acides organiques.

2. Procédé selon la revendication 1, où la SFS de l'étape b) comprend en outre les étapes de :

i) hydrolyse enzymatique avec une enzyme hydrolytique pour obtenir des saccharides fermentables ; et
ii) fermentation microbienne au moyen d'un micro-organisme qui est capable de convertir les saccharides de l'étape i) en le produit de fermentation.

3. Procédé selon la revendication 1 ou la revendication 2, où la SFS de l'étape b) est conduite dans un mode chémostat où la biomasse lignocellulosique prétraitée avec un agent alcalin est utilisée comme nutriment.

**4.** Procédé selon l'une quelconque des revendications précédentes, où la biomasse lignocellulosique prétraitée avec un agent alcalin est ajoutée à la SFS de l'étape b) d'une manière discontinue alimentée.

**5.** Procédé selon l'une quelconque des revendications précédentes, où le prétraitement de la biomasse lignocellulosique est précédé par ou combiné/intégré avec un fractionnement mécanique de la biomasse lignocellulosique, où le fractionnement mécanique est choisi de préférence dans le groupe consistant en : broyage, raffinage mécanique et extrusion.

**6.** Procédé selon l'une quelconque des revendications précédentes, où la biomasse lignocellulosique prétraitée avec un agent alcalin est soumise à une ou plusieurs des étapes suivantes avant la SFS :

    i) une étape de refroidissement ;
    ii) une étape de lavage ; et/ou
    iii) une étape de déshydratation ;

où la déshydratation est de préférence réalisée par filtration tout en appliquant une pression à la biomasse prétraitée, où la pression appliquée est située entre 0 et environ 100 bar.

**7.** Procédé selon l'une quelconque des revendications précédentes, où la température pendant la SFS est située entre environ 20 et environ 80°C et/ou où le pH pendant la SFS est régulé entre approximativement 2,0 et approximativement 10,0.

**8.** Procédé selon l'une quelconque des revendications précédentes, où le pH pendant la SFS est régulé par addition de la biomasse lignocellulosique prétraitée avec un agent alcalin et d'un alcali et où la SFS comprend de préférence une phase de pré-hydrolyse, une phase discontinue alimentée avec régulation du pH par addition d'une biomasse lignocellulosique prétraitée avec un agent alcalin et une phase discontinue avec régulation du pH par addition d'un alcali.

**9.** Procédé selon l'une quelconque des revendications précédentes, où la biomasse lignocellulosique est choisie dans le groupe consistant en : herbe, bois, bagasse, paille, papier, matière végétale et leurs combinaisons.

**10.** Procédé selon l'une quelconque des revendications précédentes, où l'agent alcalin utilisé dans l'étape a) est choisi dans le groupe consistant en : hydroxyde de calcium ($Ca(OH)_2$), oxyde de calcium (CaO), ammoniac ($NH_3$), hydroxyde de sodium (NaOH), carbonate de sodium, hydroxyde de potassium (KOH), urée et leurs combinaisons.

**11.** Procédé selon l'une quelconque des revendications 2 à 10, où l'enzyme hydrolytique de l'étape b) est choisie dans le groupe consistant en : préparations de cellulase, préparations d'hémicellulase, cellobiase, préparations de xylanase, amylase et pectinase.

**12.** Procédé selon l'une quelconque des revendications précédentes, où l'acide organique est choisi dans le groupe consistant en : acide lactique, acide citrique, acide itaconique, acide succinique, acide fumarique, acide glycolique, acide pyruvique, acide acétique, acide glutamique, acide malique, acide maléique, acide propionique, acide butyrique, acide gluconique et leurs combinaisons.

**13.** Procédé selon l'une quelconque des revendications précédentes, où le microorganisme est une bactérie, un champignon, une Archaea ou une algue, où le microorganisme est de préférence choisi parmi *espèces de Acetobacter, Bacillus coagulans, B. racemilacticus, B. laevolacticus, Corynebacterium glutamicum, Escherichia coli, espèces de Gluconobacter, espèces de Pseudomonas, bactéries lactiques, Rhizopus oryzae, Aspergillus niger, Aspergillus terreus* et *Saccharomyces cerevisiae.*

**14.** Réacteur comprenant un récipient pour le prétraitement avec un agent alcalin d'une biomasse lignocellulosique relié à un fermenteur pour la saccharification et la fermentation simultanées (SFS) de la biomasse lignocellulosique prétraitée avec un agent alcalin, où le réacteur est destiné à être utilisé dans le procédé selon l'une quelconque des revendications 1 à 13, et où :

    1) le récipient comprend :

        i) un dispositif mélangeur ;

ii) un dispositif chauffant ; et

iii) éventuellement, un moyen pour la pré-extraction des composants solubles de la biomasse lignocellulosique ;

2) le fermenteur comprend :

i) une régulation automatique du pH ; et

ii) une entrée pour une biomasse lignocellulosique prétraitée avec un agent alcalin pouvant être obtenue par la revendication 1 étape a) depuis le récipient, qui est régulée par la régulation automatique du pH,

où le moyen de liaison entre le récipient et le fermenteur est de préférence une pompe, de préférence encore un alimenteur à vis pour permettre l'alimentation automatique du fermenteur en biomasse lignocellulosique prétraitée avec un agent alcalin ;

où le fermenteur comprend de préférence un ou plusieurs des suivants :

a) une entrée pour l'alimentation automatique en un agent alcalin, qui est régulée par la régulation automatique du pH ;

b) une entrée pour une enzyme, un microorganisme et/ou un acide ou une base ;

c) une sortie pour le prélèvement d'échantillons et/ou la surveillance ; et/ou

d) une régulation automatique de la température

e) un ensemble agitateur.

**15.** Utilisation du réacteur selon la revendication 14 pour la production d'un acide organique à partir d'une biomasse lignocellulosique selon le procédé des revendications 1-13.

# Fig 1

*Fig 2a*

*Fig 2b*

*Fig 3a*

*Fig 3b*

*Fig 4a*

*Fig 4b*

*Fig 4c*

Time (h)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2004063382 A

### Non-patent literature cited in the description

- **CLAASSEN et al.** *Microbiol. Biotechnol.,* 1999, vol. 52, 741-755
- **MOSIER et al.** *Bioresour. Technol.,* 2005, vol. 96, 673-686
- **CHANG et al.** *Appl. Biochem. Biotechnol.,* 1998, vol. 74, 135-159
- **PATEL et al.** *Biotechnol. Prog.,* 2005, vol. 21, 1453-1460
- **VAN DEN OEVER et al.** *J. Mater. Sci.,* 2003, vol. 38, 3697-3707
- **KABEL et al.** *Biotechnol. Bioeng.,* 2006, vol. 93 (1), 56-63
- **MAAS et al.** *Appl. Microbiol. Biotechnol.,* 2006, vol. 72, 861-868
- **PALMQVIST et al.** *Biotechnol. Bioeng.,* 1999, vol. 63 (1), 46-55
- **KAAR ; HOLTZAPPLE.** *Biomass and Bioenergy,* 2000, vol. 18, 189-199
- **GUEBEL.** *Biotechnol. Lett.,* 1992, vol. 14 (12), 1193-1198
- **ÅKERBERG ; ZACCHI.** *Bioresour. Technol.,* 2000, vol. 75, 119-126